# EUROPEAN PATENT APPLICATION

(11) **EP 0 897 691 A2**
(43) Date of publication of application: **24.02.1999**
(21) Application number: 98115291.1
(22) Date of filing: 13.08.1998
(51) Int. Cl.: A61B 5/00

(54) **Method of and apparatus for organism measurement**

(30) Priority: 22.08.1997 JP 242105/97; 04.03.1998 JP 71487/98
(71) Applicant: Kyoto Dai-ichi Kagaku Co., Ltd., Kyoto-shi, Kyoto 601 (JP); Kurashiki Boseki Kabushiki Kaisha, Kurashiki-shi Okayama 710 (JP)
(72) Inventor: Kexin, Xu, Higashi Kujo, Minami-ku, Kyoto 601 (JP)
(74) Representative: Schoppe, Fritz, Dipl.-Ing.

(57) **Abstract**

A current substance temperature of a measured portion or a current surrounding temperature is detected before measurements and the detected current substance temperature (t₀), the detected current surrounding temperature (T₀) or a previously obtained optimum heat conduction temperature (T₁) of a measured object (12) is regarded as a target temperature level for controlling the temperature of a probe (14) with a temperature control system (16). The probe (14) is brought into contact with the measured object (12) when its temperature reaches the target temperature level, for irradiating the measured object (12) with light from an optical fiber member (15) embedded into the probe (14) and measuring a physical quantity of the measured object (12).

## Description

### BACKGROUND OF THE INVENTION

### Field of the Invention

The present invention relates to a contact type non-invasive measuring method for making measurements by bringing a probe for physical quantity measurements into contact with an organism and an apparatus therefor. Specifically, it relates to a non-invasive measuring method for irradiating a human body with light of a near infrared region and measuring a physical quantity in the human body such as hematic glucose concentration or hemal oxygen saturation for example, through output light (including all light outputted from an organism such as transmitted light, scattered light or reflected light) from the human body resulting from the light and an apparatus employed therefor.

### Description of the Prior Art

A non-invasive measuring method of irradiating an organism with light and measuring hemal oxygen saturation or a blood-sugar level from resultant output light has been undertaken in the field of clinical tests.

In order to measure a physical quantity such as hemal component concentration for example, in a living body such as a human body, it is necessary to attain interaction with the body surface of the measured person in some form through a measuring apparatus. When making measurements through the principle of absorption of light for example, it is necessary to irradiate the human body with light. Some interaction with the measured object also exists when employing a measuring method other than the irradiation with light.

In a contact type measuring method directly bringing a probe into contact with a measured object, it is conceivable that a measurement error is increased unless influence exerted on the measured object by the probe coming into contact with the same is suppressed to a minimum. Particularly, in the case of a living object thing such as a human body, physical or mental influence exerted by the measuring method varies with the difference between an equilibrium state of the human body and the state of the probe. As for the difference between the states, it is conceivable that an adjusting function for changing to a new equilibrium state acts to change the measured value regardless of whether the human himself is conscious or unconscious. It is conceivable that disturbance is caused in the measurement of the physical quantity through the adjusting process, to exert influence on the measured value.

In more concrete terms, in the case of a contact type measurement bringing the probe into contact with a measured person, the temperature of a probe is different from both the surrounding temperature and temperature of a measured person, i.e., a human body. When the probe comes into contact with the human body in this state, it is conceivable that an adjusting function of temperature-controlling the measured portion acts through the mechanism of heat conduction or the like in the humanbody, in order to attain equilibration in the new surroundings. Furthermore, the surface temperature of the human body varies depending on the ability of temperature control, regardless of the surroundings. It is conceivable that the process of heat conduction as well as the time required for stabilization varies with the temperature difference between the human body and the probe coming into contact with the measured portion and the surroundings. Furthermore, it is also conceivable that, if the temperature of the probe coming into contact with the human body is different from the surrounding temperature which is familiar to him, he may feel cold and be mentally influenced, resulting in conscious or unconscious influence on physiological phenomena.

In the case of a human body, such influence is quantitatively unknown under present circumstances. In other words, it is extremely difficult to grasp what is influenced internally and what kind of change is mentally caused by a mechanism such as heat conduction for example, and it can be said that this is rather inconceivable under present circumstances. Specifically, when an optical non-invasive measurement is made for measuring the concentration of a minor component such as hemal glucose concentration for example, signals are extremely weak by nature and hence the influence exerted by the temperature difference deteriorates a signal-to-noise ratio and makes extraction of the signal further difficult. This is considered as being one of the factors hindering improvement in the accuracy of non-invasive glucose measurement at present.

Table 1 shows exemplary measured data on fat of a human hand.

**(Table 1)**

| Surface Temperature of Hand (°C) | Fatty Ratio (%) | Fat Quantity (Kg) | Tissue Quantity (Kg) |
|---|---|---|---|
| 25.5 | 19.8 | 14.8 | 60.1 |
| 32.7 | 15.8 | 11.8 | 63.1 |
| 34.0 | 17.3 | 12.9 | 62.0 |
| 36.0 | 19.0 | 14.2 | 60.7 |

The above results have been obtained by forcibly changing the temperature of the hand. It is hard to believe that the fatty ratio simply varies by the temperature of the hand. Therefore, it is proper to consider that the dispersion of the measured data of the fatty ratio is influenced by biological change resulting from forcible temperature control serving as disturbance, in addition to change of the absorption coefficient resulting from the change of the temperature.

PCT International Laying-Open Gazette No. WO95-06431 describes means of increasing the blood flow of a part in contact with a measuring apparatus, by increasing the temperature of the measuring apparatus beyond the body temperature on page 24. However, when the temperature is forcibly increased in such a manner, the organism is changed from a general stable state to a different state as understood from the fact that the blood flow increases, and this is unpreferable in view of reproducibility of measured data.

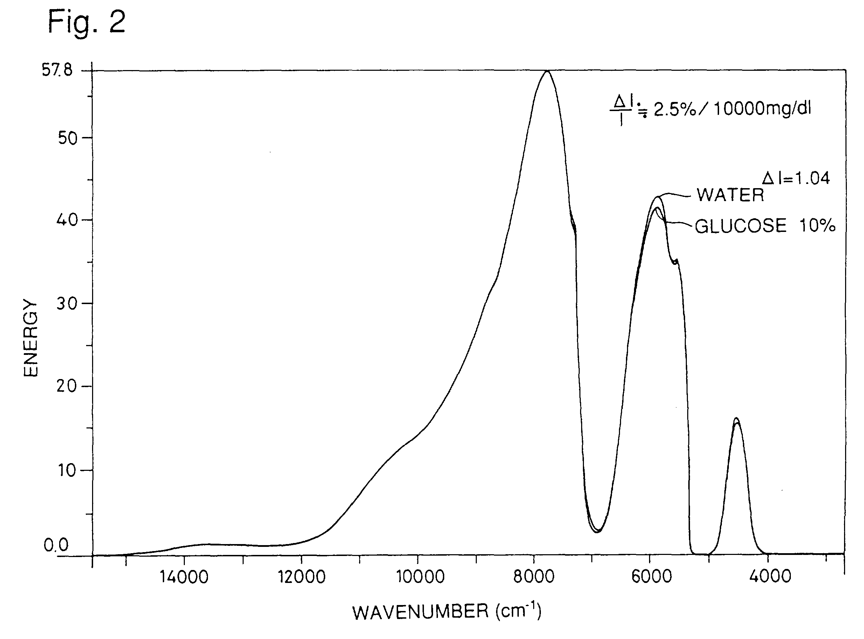

It is known that an absorption coefficient for light varies with the temperature. As for water, J. Chem. Phys. 68(10), 15 May 1978, for example, shows change of an absorption spectrum of water in Fig. 2.

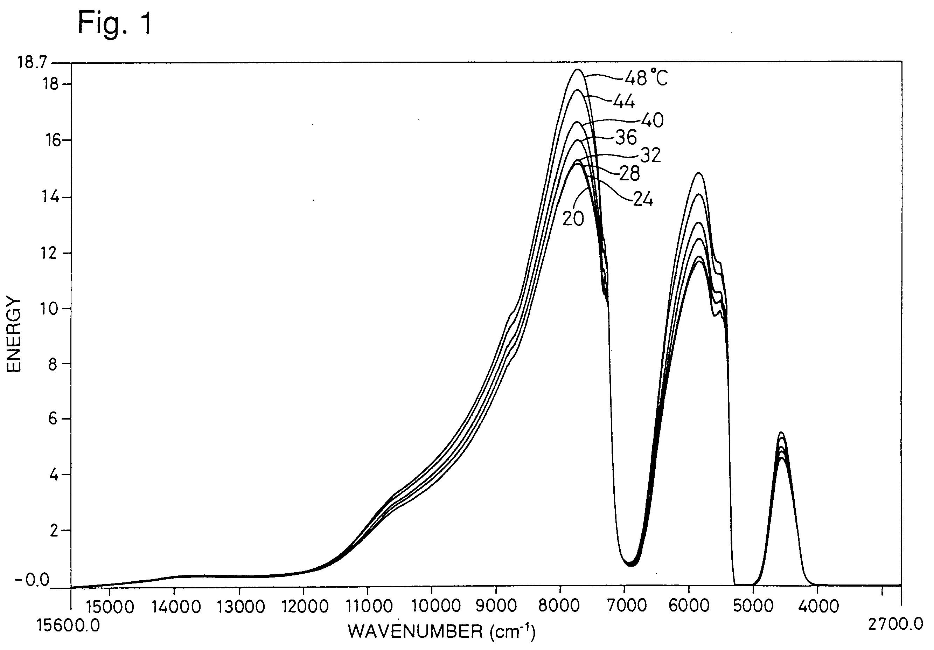

Fig. 1 shows data obtained by investigating how much light absorption of water varies with the temperature. A cell having an optical path length of 1 mm has been employed for measuring an absorption spectrum of water by changing a water temperature from 20°C to 48°C and indicating the results in transmission energy. For example, an energy change ratio at a wavenumber of 6000 cm⁻¹ is 0.93 %/°C.

Conversely, Fig. 2 shows results of a transmission absorption measurement of a glucose solution performed for inferring how much light quantity change results from an optical measurement of a blood-sugar level. For example, relative change of light intensity corresponding to a concentration change of 10000 mg/dl is about 2.5 % at an absorption wavenumber of 1679 nm (about 6000 cm⁻¹) for glucose. Since the change range of glucose concentration in a biological change range of a human body is 400 mg/dl, it comes to that there is only a relative change of light quantity of 0.1 % in terms of relative change of light absorption intensity by glucose. In other words, it comes to that relative light quantity change of absorption resulting from a temperature change of 1°C is 9.3 times that through a general change range of glucose concentration.

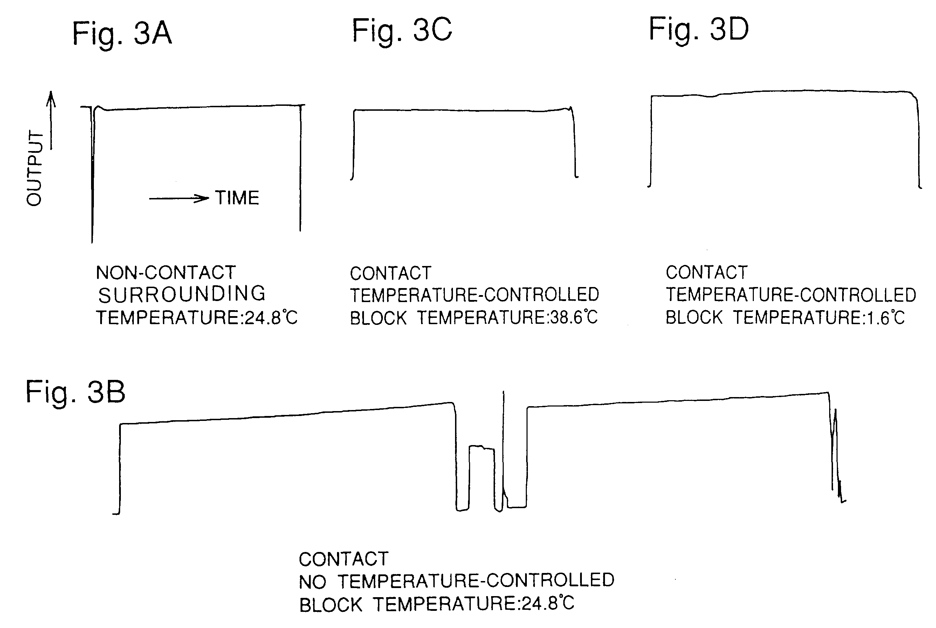

Figs. 3A to 3D show results of scattered light quantity changes based on the temperature change of an organism, under the same measuring scale. A finger of a hand was irradiated with light for measuring scattered light quantities.

Fig. 3A shows a result in the case of arranging the finger in the air allowing a non-contact state. In this case, the surrounding temperature was 24.8°C. At this time, the finger was in an equilibrium state with the surrounding temperature, and heat exchange with the surroundings was slow. Fluctuation of the scattered light quantity is small.

Fig. 3B shows a result of bringing the finger into contact with a metal block of 24.8°C, which was not temperature-controlled. In this case, heat flew from the finger to the metal block, to change the temperature of the finger. While the measurement was made twice, fluctuation of the scattered light quantity was large in both cases.

Fig. 3C shows a result of bringing the finger into contact with a metal block of 38.6°C, which was temperature-controlled. In this case, movement of heat was small since the temperature of the metal block was close to that of the finger. The scattered light quantity fluctuation is small but larger than the case in Fig. 3A.

Fig. 3D shows a result of bringing the finger into contact with a metal block of 1.6°C, which was temperature-controlled. In this case, movement of heat was large due to a large temperature difference between the metal block and the finger. Scattered light quantity fluctuation larger than that in Fig. 3C is observed.

In this manner, it has been proved that an optical measured value varies with temperature differences between a measured object and a probe or the like which is brought into contact with the same, in the case of making non-invasive measurements in a contact state.

### SUMMARY OF THE INVENTION

The object of the present invention is to attain stabilization of measured data by reducing influence exerted on an organism, which is a measured object, by contact with a probe for measurement through a contact type non-invasive measuring method.

The present invention is directed to a contact type non-invasive measuring method for making measurements by bringing a probe for physical quantity measurement into contact with an organism. According to a first aspect of the present invention, the method comprises steps of detecting the temperature of a measured portion of the organism immediately before the measurement and regarding the same as a current substance temperature, regarding the current substance temperature as a target temperature level for the probe and controlling the temperature of the probe, and bringing the probe into contact with the measured portion of the organism for making physical quantity measurements while continuing temperature control so that the temperature of the probe is steady after reaching the current substance temperature.

According to a second aspect of the present invention, the method comprises steps of detecting the surrounding temperature for a measured portion immediately before a measurement and regarding the same as a current surrounding temperature, regarding the current surrounding temperature as a target temperature level for the probe, and bringing the probe into contact with the measured portion of the organism for making physical quantity measurements, while continuing temperature control so that the temperature of the probe is steady after reaching the current surrounding temperature.

According to a third aspect of the present invention, the method comprises steps of previously obtaining a temperature stabilizing a measured physical quantity of a measured portion of the organism as a physical quantity measuring temperature, regarding the physical quantity measuring temperature as a target temperature level for the probe, and bringing the probe into contact with the measured portion of the organism for making physical quantity measurements, while continuing temperature control so that the temperature of the probe is steady after reaching the physical quantity measuring temperature.

The physical quantity measuring temperature is a surrounding temperature corresponding to a state most stabilizing the measured portion (internal and external). Since it is conceivable that the physical quantity measuring temperature varies by the measured portion or a measured person, it is necessary to previously obtain each physical quantity measuring temperature in response to the measured portion or the measured person. The physical quantity measuring temperature can be obtained by irradiating an organism with light at each temperature value while temperature-controlling the measured portion of a human body at different target temperature levels and measuring the light quantity stability of outgoing light from the organism resulting from the light. A temperature stabilizing measured data is previously obtained and registered as the physical quantity measuring temperature for the measured object or the measured portion.

Upon setting the physical quantity measuring temperature, the following two points must be taken into consideration:
(1) Assuming that the measured object is a human body, a temperature gradient from the surface of the human body to the interior varies whether measured objects of different temperatures are temperature-controlled to the same target temperature level, or those of the same temperature are temperature-controlled to different target temperature levels, and consequently change of a scattered state of light takes place by physical influence of a tissue resulting from differences between volumes or the like, and a measurement result varies to exert influence on measurement accuracy. In order to suppress this change, it is necessary to perform temperature control in a direction for settling the temperature gradient. If stability of a measurement result is excellent when the temperature difference between the target temperature level and the temperature of the measured object is 3°C for example, the target temperature level is set at 39°C if the temperature of the measured object is 36°C, while the target temperature level is set at 33°C if the temperature of the measured object is 30°C for performing temperature control.
(2) Even at a temperature which is in an equilibrium state with the surroundings, a biologically instable state may result if the temperature gradient from the surface to the interior is improper, to possibly deteriorate stability of measured data and exert influence on measurement accuracy.

Therefore, it is necessary to select a temperature stabilizing measurement data by exclusively taking the above items (1) and (2) into consideration when setting the physical quantity measuring temperature.

The present invention is also directed to a measuring apparatus for carrying out the aforementioned method. According to a fourth aspect of the present invention, the measuring apparatus comprises a probe coming into contact with an organism for measuring a physical quantity in a non-invasive manner, a first temperature sensor detecting the temperature of a measured portion of the organism or its periphery, heating/cooling means provided on the probe, a second temperature sensor for probe temperature detection provided on the probe, and a temperature controller regarding the temperature detected by the first temperature sensor as a target temperature level for the probe and controlling the temperature of the probe so that the temperature detected by the second temperature sensor reaches the target temperature level.

According to a fifth aspect of the present invention, the measuring apparatus previously stores a temperature stabilizing a physical quantity measurement value of a measured portion of an organism as a physical quantity measuring temperature, while the measuring apparatus according to the fourth aspect of the present invention detects the temperature of the measured portion of the organism or its periphery with the first temperature sensor. The measuring apparatus regards the physical quantity measuring temperature as a target temperature level for a probe, and controls the temperature of the probe to reach the target temperature level.

The measuring apparatus according to the present invention detects the temperature of the measured portion immediately before a measurement or the surrounding temperature for the measured portion, or previously obtains and stores the physical quantity measuring temperature in case of making a non-invasive measurement in a contact type by bringing the probe into contact with the organism after its temperature reaches the target temperature level, whereby influence exerted on the organism serving as a measured object by contact with the probe is reduced and measured data is stabilized.

The foregoing along with other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a spectral diagram showing variations of an absorption coefficient of water with the temperature.
Fig. 2 illustrates a transmission absorption spectrum of a glucose solution.
Figs. 3A to 3D illustrate results of scattered light quantity change based on temperature change of an organism.
Figs. 4A, 4B and 4C illustrate steps of detecting a current substance temperature of a measured portion, or a current surrounding temperature before measurement, controlling the temperature of a probe to reach the temperature detected in Fig. 4A or a previously obtained physical quantity measuring temperature, and making a physical quantity measurement respectively.
Fig. 5 schematically illustrates the present invention in the case of employing water as a probe.
Fig. 6 schematically illustrates a temperature control method in an embodiment employing a solid probe.
Fig. 7 schematically illustrates a temperature control method in an embodiment employing a liquid probe.
Fig. 8 is a block diagram showing a measuring apparatus according to the present invention.
Fig. 9 is a block diagram showing an embodiment comprising an integrating sphere type probe.

### DESCRIPTION OF THE PREFERRED EMBODIMENTS

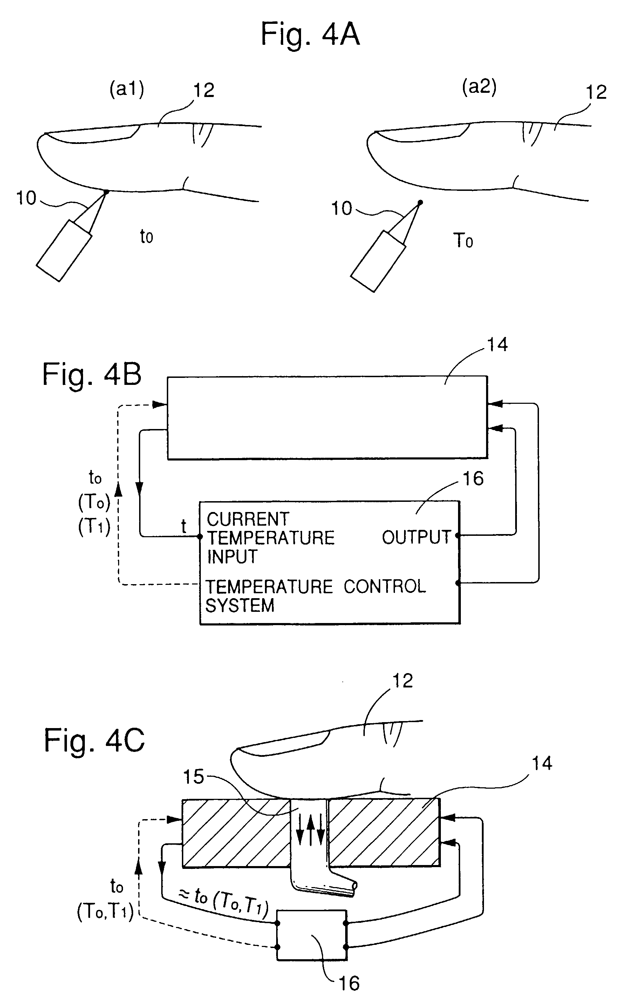

Figs. 4A to 4C illustrate such an embodiment that an optical fiber member for example, is embedded into a solid block of a metal to serve as a probe for measuring a physical quantity. A measured portion of an organism is irradiated with light from the optical fiber member so that outgoing light from the measured portion enters the optical fiber member again and is detected.

Fig. 4A shows a step "a1" or "a2" of detecting a current substance temperature of the measured portion or a current surrounding temperature before measurement. In step "a1" which is adapted to detect the current substance temperature, a temperature sensor 10 is brought into contact with a finger 12 serving as the measured object, immediately before a physical quantity measurement, to detect the temperature of the finger 12 and regarding the same as a current substance temperature (t₀). On the other hand, in step "a2" which is adapted to detect the current surrounding temperature, the temperature sensor 10 detects the surrounding temperature around the finger 12 immediately before a measurement of the physical quantity, for regarding the same as a current surrounding temperature (T₀).

Fig. 4B shows a step of controlling the temperature of a probe 14 to a target temperature level of the current substance temperature (t₀)or the current surrounding temperature (T₀) detected in step "a1" or "a2" in Fig. 4A or a previously obtained physical quantity measuring temperature (T₁) of the measured object. A temperature control system 16 controls the temperature of the probe 14 to the target temperature level of the current substance temperature (t₀), the current surrounding temperature (T₀) or the physical quantity measuring temperature (T₁).

Fig. 4C shows a step of measuring the physical quantity by bringing the probe 14 into contact with the measured object 12 when its temperature reaches the target level, irradiating the measured object 12 with light from an optical fiber member 15 which is embedded in the probe 14 and measuring the physical quantity of the measured object 12.

The temperature control system 16 comprises a temperature controller, a driver and a heating/cooling element.

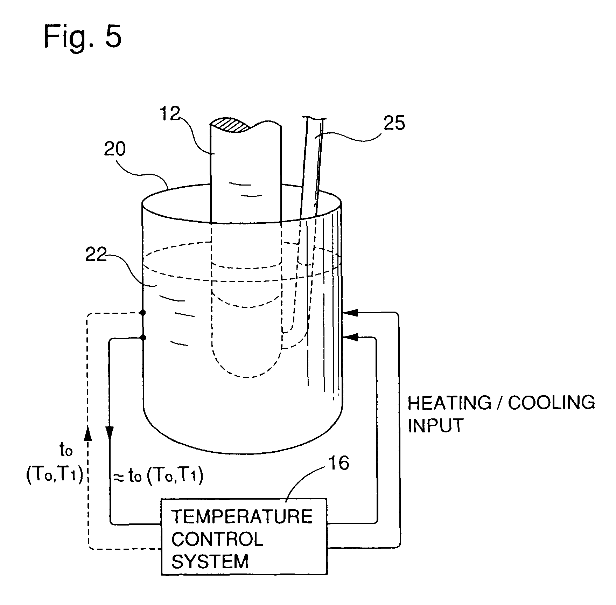

Fig. 5 shows an embodiment employing a liquid such as water for example, as a probe. A container 20 stores water 22, for dipping the finger 12 serving as the measured object therein. The current substance temperature (t₀) or the current surrounding temperature (T₀) shown in Fig. 4A or the physical quantity measuring temperature (T₁) is employed as the target temperature level of the temperature control system 16, for controlling the temperature of the water 22 stored in the container 20 to reach the target temperature level. When the temperature of the water 22 reaches the target temperature level, the finger 12 is irradiated with light from an optical fiber probe 25 for an optical measurement in the water 22, to measure resulting output light.

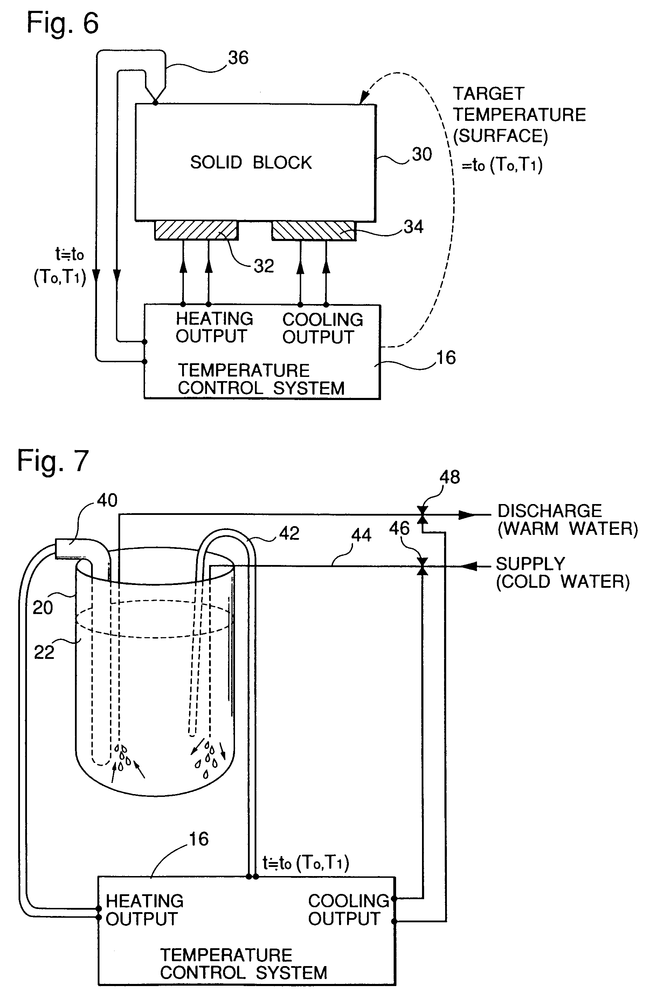

Fig. 6 shows a concrete embodiment of the solid probe 10 shown in Fig. 4C. A heating Peltier element 32 and a cooling Peltier element 34 are provided on a heat-conductive metal block 30. The temperature control system 16 controls heating and cooling by the Peltier elements 32 and 34 through its outputs. A temperature sensor 36 is mounted on the block 30, and a current is fed to either Peltier element 32 or 34 so that the temperature of the block 30 reaches a target temperature level.

In the Peltier element 32 or 34, heating and cooling can be switched depending on the energization direction of the current. Therefore, the Peltier elements 32 and 34 can be replaced with a single Peltier element in the embodiment shown in Fig. 6.

Fig. 7 shows a concrete example of a temperature control method for a liquid employed as a probe as shown in Fig. 5. A heater 40 and a temperature sensor 42 are dipped in water 22, which is stored in a container 20, so that a cooling water supply path 44 can supply cold water through a switching valve 46 for cooling. The water 22 is discharged from the container 20 through a switching valve 48, in order to adjust the water quantity in the container 20.

The water temperature is controlled on the basis of an output of the temperature sensor 42 to reach the current substance temperature (t₀), the current surrounding temperature (T₀) or the physical quantity measuring temperature (T₁) which is inputted into the temperature control system 16. The quantity of a current supplied to the heater 40 is controlled for heating, the valve 46 is opened for supplying the cold water for cooling, while the valve 48 adjusts the water quantity.

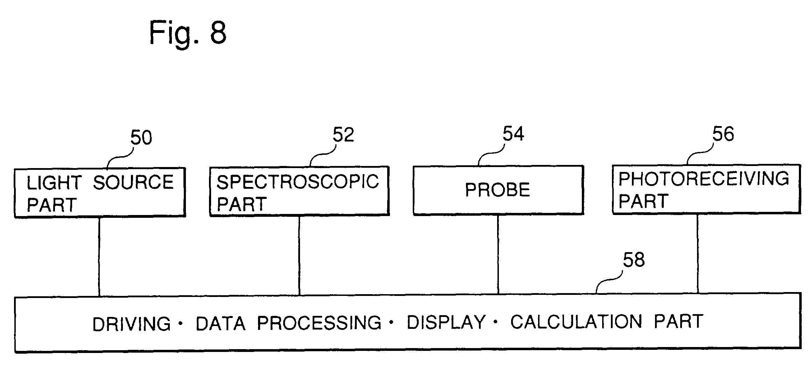

Fig. 8 schematically shows a measuring apparatus according to the present invention.

A spectroscopic part 52 selects a prescribed wavelength from light emitted from a light source part 50 comprising a halogen lamp for example. The spectroscopic part 52 is prepared from an Acousto-Optic Tunable (AOTF) for example. The AOTF is a spectroscopic element comprising an acoustic wave transducer on an acousto-optic crystal, which can perform spectroscopic analysis by changing an acoustic wave frequency (radio frequency (RF)) supplied from the acoustic wave transducer to the acousto-optic crystal. A probe 54 is adapted to irradiate a measured portion with wavelength-selected light. The probe 54 comprises a set probe having a space of the same shape as the measured portion for example, so that the measured portion is fitted in and fixed to the space, and a measuring probe consisting of an optical fiber member which is mounted on the set probe for irradiating the measured portion with light. A photoreceiving part 56 employs a photoreceptor PbS of an electronic cooling system for example, for detecting output light from the measured portion received by the probe 54.

The probe 54 comprises Peltier elements for heating and cooling and a temperature sensor similarly to the block 30 shown in Fig. 6, so that heating and cooling are controlled through outputs of a temperature control system.

A driving · data processing · display and calculation part 58 comprises a data processing part, a calculation part and a display. The data processing part electrically drives the light source part 50, the spectroscopic part 52 and the photoreceiving part 56, controls the temperature of the probe 54, and performs necessary data preprocessing on data detected by the photoreceiving part 56. The calculation part performs multivariate analysis of the processed data and calculates blood-sugar concentration, for example. The display outputs and displays the measurement result of the calculated blood-sugar concentration or the like.

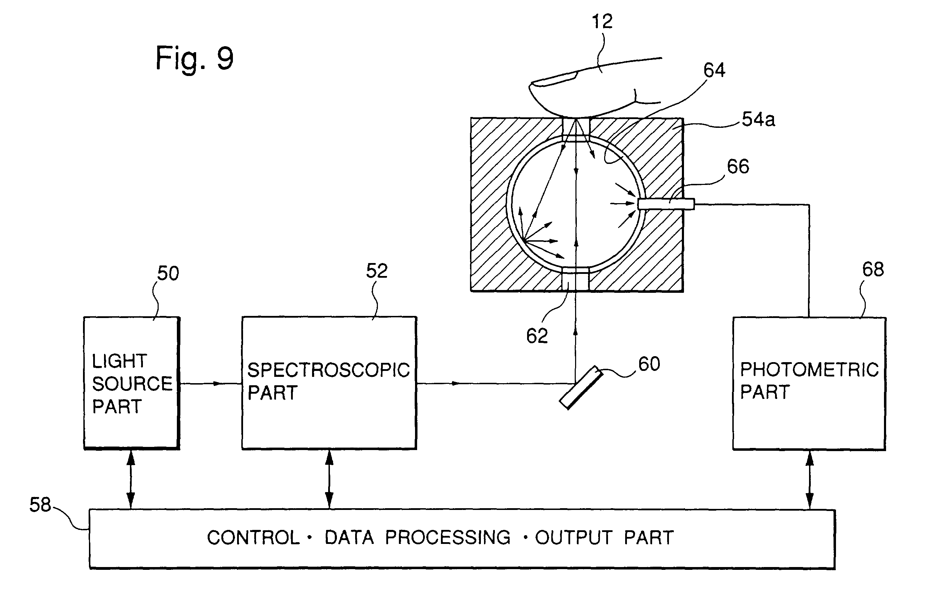

Fig. 9 shows an embodiment comprising an integrating sphere type probe 54a. The integrating sphere probe 54a comprises an inlet port 62 for receiving light spectroscopically separated in a spectroscopic part 52 and reflected by a mirror 60 on a part thereof, and an opening is formed on a position of an integrating sphere 64 opposed to the inlet port 62 so that a finger 12 being the measured object can be pressed against the opening. The integrating sphere 64 is further provided with a detector 66 for photodetection, so that light received and detected by the detector 66 is converted to an electric signal and fed to a photometric part 68.

The probe 54a is made of a metal and provided with Peltier elements (not shown) and a temperature sensor (not shown) which are similar to those shown in Fig. 6 for temperature control, and a temperature control system controls the temperature of the probe 54a to reach the current substance temperature (t₀), the current surrounding temperature (T₀) or the physical quantity measuring temperature (T₁).

When such an integrating sphere probe 54a is employed, it is possible to efficiently collect and receive outgoing light from a measured portion, and consequently this is suitable for detecting a weak signal such as that in a blood-sugar level measuring system.

Although the present invention has been described and illustrated in detail, it is clearly understood that the same is by way of illustration and example only, and is not to be taken by way of limitation as the spirit and scope of the present invention is limited only by the terms of the appended claims.

## Claims

1. A contact type non-invasive measuring method for making measurements by bringing a probe(14) for physical quantity measurement into contact with an organism(12), characterized by comprising the steps of:
setting a target temperature level for said probe(14);
controlling the temperature of said probe(14) to reach said target temperature level; and
bringing said probe(14) into contact with a measured portion of said organism(12) for performing physical quantity measurement while continuing temperature control for settling the temperature of said probe(14) at said target temperature level after reaching said target temperature level.

2. The measuring method in accordance with claim 1;
detecting the temperature of said measured portion of said organism(12) as a current substance temperature(t₀) immediately before measurements and regarding the temperature(t₀) as said target temperature level.

3. The measuring method in accordance with claim 1;
detecting the surrounding temperature around said measured portion of said organism(12) as a current surrounding temperature(T₀) immediately before measurements and regarding the temperature(T₀) as said target temperature level.

4. The measuring method in accordance with claim 1;
previously detecting a physical quantity measuring temperature(T₁) stabilizing a physical quantity measuring value of said measured portion of said organism(12) and regarding the temperature(T₁) as said target temperature level.

5. The measuring method in accordance with claim 4;
previously obtaining each value of said physical quantity measuring temperature(T₁) in response to said measured portion or a measured person.

6. The measuring method in accordance with claim 4;
obtaining said physical quantity measuring temperature(T₁) as a temperature stabilizing measured data by irradiating said organism with light at each temperature value while temperature-controlling said measured portion of said organism(12) with several types of different target temperature levels and measuring light quantity stability of outgoing light from said organism resulting from said light.

7. An organism measuring apparatus comprising:
a probe(14, 22, 30) coming into contact with an organism(12) and measuring a physical quantity in a non-invasive manner;
a heating/cooling apparatus(32, 34, 40) provided on said probe;
a temperature sensor(36, 42) for probe temperature detection provided on said probe(14, 22, 30); and
a temperature controller(16) controlling the temperature of said probe(14, 22, 30) with said heating/cooling apparatus(32, 34, 40) so that the temperature detected by said temperature sensor(36, 42) reaches a target temperature level.

8. The organism measuring apparatus in accordance with claim 7;
further comprising another temperature sensor(10) detecting the temperature of a measured portion of said organism(12) or its periphery, for regarding the temperature detected by said another temperature sensor(10) as said target temperature level for said probe(14, 22, 30).

9. The organism measuring apparatus in accordance with claim 7;
further comprising a memory device previously storing a temperature stabilizing a physical quantity measured value of a measured portion of said organism(12) as a physical quantity measuring temperature(T₁), for regarding said physical quantity measuring temperature(T₁) stored in said memory device as said target temperature level for said probe(14, 22, 30).

10. The organism measuring apparatus in accordance with claim 7; wherein
said probe(14) is prepared by embedding an optical fiber member(15) for measuring said physical quantity into a metal block(30), for irradiating a measured portion of said organism(12) with light from said optical fiber member(15) so that outgoing light from said measured portion enters said optical fiber member(15) again and is detected.

11. The organism measuring apparatus in accordance with claim 10; wherein
said metal block(30) of said probe(14) is made of a heat-conductive metal and provided with a Peltier element(22, 34), so that said temperature controller(16) controls heating and cooling of said metal block(30) through said Peltier element(32, 34).

12. The organism measuring apparatus in accordance with claim 7; wherein
said probe is prepared by providing an optical fiber member(25) for an optical measurement in a temperature-controlled liquid(22), so that a measured object(12) is dipped in said liquid(22) and irradiated with light from said optical fiber member(25) and output light therefrom is measured.

13. The organism measuring apparatus in accordance with claim 12; wherein
a heater(40) and said temperature sensor(42) are dipped in said liquid(22) of said probe, and a cooling water supply path(44) is provided for supplying cold water through a switching valve(46) for cooling.

14. The organism measuring apparatus in accordance with claim 7; wherein
a light inlet port(62) is provided on a part of an integrating sphere type metal probe(54a), an opening against which a measured object(12) is pressed is provided on a position opposed to said inlet port(62) and a detector(66) for photodetection is provided in another position, while a Peltier element for temperature control and said temperature sensor are provided on said metal probe(54a).
